# EUROPEAN PATENT SPECIFICATION

(11) **EP 3 938 004 B1**
(45) Date of publication and mention of the grant of the patent: **03.05.2023**
(21) Application number: 20711851.4
(22) Date of filing: 11.03.2020
(51) Int. Cl.: A61F 13/00, A61M 1/00

(54) **NEGATIVE PRESSURE TREATMENT ARRANGEMENT**
UNTERDRUCKBEHANDLUNGSANORDNUNG
CONFIGURATION DE TRAITEMENT PAR PRESSION NÉGATIVE

(30) Priority: 12.03.2019 DE 202019101382 U
(43) Date of publication of application: 19.01.2022
(73) Proprietor: Lohmann & Rauscher GmbH, 1140 Wien (AT)
(72) Inventor: Loske, Gunnar, 22926 Ahrensburg (DE)
(74) Representative: Seranski, Klaus
(86) International application number: PCT/EP2020/056509
(87) International publication number: WO 2020/182887

(56) References cited:
- EP-A1- 2 563 422
- US-A1- 2012 016 321
- US-A1- 2016 354 253
- US-A1- 2018 353 662

## Description

The invention relates to a negative pressure treatment arrangement for wound treatment as specified in the pre-characterizing portion of claim 1.

At the end of an operation, the general aim is to use stitches, clips or adhesive to effect a primary wound closure. Exceptions to this are infected wounds, as in the case of a perianal abscess for example, or heavily contaminated wounds. The wounds are then left to allow secondary wound healing to occur, whereby healing takes the form of a formation of granulation tissue from the base of the wound, or a secondary wound closure is performed.

The same applies to wounds resulting from postoperative wound infections. These occur with a cumulative incidence ranging from 1% in endoprosthetics to 9.5% in colorectal surgery, depending on the operation performed.

In addition to the nature and length of the operation, innate patient factors such as a high BMI, advanced age and comorbidities increase perioperative risk. The costs of treating these wounds are estimated at roughly 20% of the total treatment costs for problematic wounds.

The treatment and avoidance of post-operative wound infections is therefore the subject of current surgical research. The use of negative pressure treatment is widespread in the area of secondary wound healing. Cited advantages over conventional wound treatment include improved blood circulation, closing of the wound edges and evacuation of the exudate.

Current, largely retrospective studies show significantly positive effects in connection with negative pressure wound therapy with reduced wound infection rates, particularly in oncology patients following a laparotomy with contaminated laparotomy wounds following a dissection and plastic surgery. In the case of known negative pressure treatment arrangements for wound treatment, a polyurethane foam is placed over the wound and a negative pressure is applied to the wound via the polyurethane foam.

In the case of a negative pressure treatment arrangement for wound treatment in accordance with WO 2014/044400, a film with one open-cell side is used. The film can have an adhesive along edges at least and be stuck onto a wound or onto the skin surrounding the wound. However, wound healing still is compromised in many cases if the known wound treatment arrangements are used.

Negative pressure treatment arrangements are disclosed in WO 2011/135287 A1, EP 2563422 A1, US 2012/016321 A1, US 2016/0354253 A1, and US 2018/0353662 A1. Arrangements according to the precharacterizing portion of claim 1 are disclosed in US 2018/0353662 A1.

In view of these problems in the prior art, the object of the invention is to specify negative pressure treatment arrangements for wound treatment which can be used to ensure wound treatment and wound healing with as few complications as possible.

According to the invention, this object is fulfilled with a negative pressure treatment arrangement for wound treatment as specified in amended claim 1.

According to the invention, the drainage film is designed in some areas at least from a transparent material. This invention is based on the finding that the deficiencies observed in conventional wound treatment are attributable to that fact that the negative pressure treatment arrangement is changed at inappropriate junctures. This can be the case, for example, if healing of the wound is not yet sufficiently advanced, but also if the negative pressure treatment arrangement as such could still be deemed to be functional, meaning that the negative pressure treatment arrangement is being changed too frequently. If the drainage film and/or the fixing film are designed in some areas at least from a transparent material, the wound healing process can be observed during negative pressure treatment and manipulation of the negative pressure treatment arrangement at the appropriate time can be ensured. In the context of the invention, the property transparent refers to the feature whereby a vague profile of the covered wound at least is visible through the drainage film and/or the fixing film. This property can also be described as translucent. The fixing film of a negative pressure treatment arrangement according to the invention may in some cases have a transparent polyurethane film. The polyurethane film is preferably coated on one side at least with an adhesive, such as a polyacrylate adhesive. A backing film can be provided on the side of the fixing film facing away from the adhesive. The backing film can be a polyester film coated with polyethylene on both sides and/or a cover paper siliconised on one side. The cover paper can be removed once the negative pressure treatment arrangement has been placed on the wound.

This invention is also based on the knowledge that conventional negative pressure treatment arrangements do not sufficiently protect the wound during negative pressure treatment and can lead to complications when changing the film with one open-cell side and/or the open-cell polyurethane foam in the course of therapy.

Within the invention, these issues are resolved by using a multi-layer drainage film which provides a padding function to protect the wound. By separating the function of the drainage film on the one hand and the function of the fixing film on the other hand, the properties of the individual elements of the wound treatment arrangement can be adapted to the required function. The drainage film can thus be designed without an adhesive and even be provided with a feature reducing the adhesion to the base of the wound, while the fixing film can be optimised in terms of the adhesion to the skin surrounding the wound and the covering of the wound space.

This allows impairments of wound healing when changing the wound treatment arrangement, as may be required during long-term treatment, to be reduced, whereby impairments of wound healing can also be avoided with the cushioning function of the multi-layer drainage film. Finally, the application of wound treatment arrangements according to the invention is simplified by the fact that the drainage film can be designed so that it is completely symmetrical if the first and second opening arrangements in the film layers delimiting the drainage space are designed identically.

The opening arrangements can be distributed over large areas of the individual film layers such that a negative pressure can be produced across large areas of the drainage space around the wound. A connection for a device to produce a negative pressure can be positioned freely on the second opening arrangement according to the respective application. This also enables an impairment of the wound healing process to be avoided. It is envisaged, for example, that the connection for the device for producing the negative pressure be provided in the area of the skin surrounding the wound, wherein the negative pressure is applied to the wound space by the drainage space.

The connection arrangement for connecting a device for producing negative pressure can comprise, for example, an open-cell sponge that can be placed on the second opening arrangement and fixed, where necessary, to the second opening arrangement with the fixing film and/or comprise a drainage hose placed inside the open-cell sponge where applicable.

Corresponding negative pressure treatment arrangements can be applied such that first the drainage film is placed on the wound such that the first opening arrangement is facing the wound, then the open-cell sponge or a flange with a fitting designed for connecting a drainage hose is placed on the second opening arrangement of the film layer facing away from the wound and finally the fixing film is applied to the arrangement prepared accordingly so that it overlaps the open-cell sponge and/or flange such that the open-cell sponge and/or flange is arranged between the fixing film and drainage film with a fixing area. The negative pressure treatment arrangement according to the invention can then be connected to a device for producing a negative pressure, such as a pump, by means of drainage hose to thus introduce negative pressure treatment.

According to the invention, the drainage film comprises at least one bulge containing the second opening arrangement, wherein the drainage film is transparent in design in some areas at least outside of this bulge at least. If the bulge containing the second opening arrangement is located next to the actual wound area during wound treatment, a clear view of the wound can thus be ensured. The sponge and/or flange can be applied outside of the wound area in the area of the bulge. By for example extending the drainage film, arranging it in a T or L shape for example, the sponge and/or flange can be applied next to the wound to thus maintain an unobscured view of the entire wound. However, the negative pressure can still be passed on in full and exudate can still be discharged fully through the drainage space of the drainage film.

In a preferred embodiment of the invention, a suction body for receiving and storing fluids from the wound is preferably located in the area of the bulge. The suction body can be provided between the fixing film and the skin surrounding the wound and/or in fluidic connection with the drainage film and may possibly only extend slightly into the wound area. If the possibly transparent dressing extends beyond the actual extent of the wound and the suction body is positioned next to the wound, it can serve as a fluid collector, for example as an exudate collector. The exudate is directed into the suction body as a result of the negative pressure applied to the negative pressure treatment arrangement according to the invention and by means of the drainage film. If the drainage film and/or the fixing film is transparent in design, it is possible to devise an arrangement whereby transparency is maintained over the wound. It is therefore possible to maintain an unobscured view of the wound even where there is a significant flow of exudate.

If a device for producing a negative pressure, such as a pump, is also incorporated into the negative pressure treatment arrangement according to the invention, this could be connected via or in the direction of flow of the exudate behind the suction body. In the case of this embodiment, for example where a pump is connected to the negative pressure arrangement according to the invention via a drainage hose, the suction body can be replaced without having to open the entire dressing over the wound if it is arranged in the area of the bulge located outside of the wound space. In other embodiments of the invention, however, a concept is adopted whereby the suction body is located in the area of the wound itself and/or an external suction body is provided, possibly in the area of the drainage hose and/or the flange used to connect the drainage hose and/or in the area of the pump.

In the case of a preferred embodiment of the invention, the depth of the drainage space in a depth direction extending perpendicular to the film layers delimiting the drainage space is chosen such that a capillary action is triggered in the bodily fluids such as exudate collected in the drainage space. This capillary action of the drainage space, which also ensures uniform production of the required negative pressure in the wound space via the first opening arrangement, firstly prevents a discharge of bodily fluids, in particular exudates, from the drainage space and secondly allows a distribution of the bodily fluids via the entire drainage surface or drainage film, without additional measures such as the provision of additional absorbent bodies being imperative. The drainage film therefore not only improves the negative pressure therapy, but also allows good exudate management across the entire wound area.

Relevant drainage films are described in EP-A-10716300, for example.

With regard to the required padding function of the drainage film, the first opening arrangement has at least one channel starting from the first film layer and proceeding towards the opposite boundary surface of the other film layer and leading into the drainage space, the wall of which channel is designed as a single piece with the film layer, and is formed by perforating the film layer, as this allows an effective cushion to be provided in the drainage space.

In the latter embodiment of the invention described, exudate management is improved particularly effectively with the cross-sectional area of the channel being reduced on a plane extending perpendicular to the depth direction, starting from the film layer and proceeding towards the opposite internal boundary surface, in particular to maintain a capillary action promoting the admission of bodily fluid to the drainage space.

In terms of both the required exudate management and the required production of a negative pressure in the wound space, at least one opening arrangement has a plurality of openings preferably arranged in a grid pattern, wherein the spacing between adjacent openings is 15 mm or less, preferably 5 mm or less, and in particular 3 mm or less. In terms of the desired padding effect, it has proven efficacious if the apertures of the openings located in one film layer are arranged in a projection along the depth direction between the apertures of the openings located in the other film layer.

This arrangement of the openings in wound covers in accordance with the invention can be used to particular advantage for openings formed by channels if the wound cover is used in conjunction with a negative pressure source used to suck the exudate from the wound area, in order to thus prevent the entire structure from collapsing when negative pressure is applied. In this context, it has proven particularly beneficial if at least one channel forming an opening in a web-shaped element extends in the depth direction over 50% or more of the full depth of the drainage space.

To ensure the required permeability of the web-shaped elements, it has proven efficacious if the aperture areas of the individual openings facing the drainage space are 0.1 mm² or more, in particular 0.5 mm² or more, particularly preferably 1 mm² or more on a plane running perpendicular to the depth direction. The required capillary action can be achieved while avoiding a backflow of fluid from the drainage space to the wound space if the aperture area of the openings is 5 mm² or less, in particular 4 mm² or less, particularly preferably 3 mm² or less.

As already explained above, the channels forming the openings in the web-shaped elements can be formed by means of perforations in the web-shaped elements or film layers. In this context, it has proven beneficial for the maintenance of a smooth surface facilitating the adhesion of tissue or cells to the base of the wound and the production of an effective negative pressure in the surrounding organs if sections at least of the channel wall are designed in an arc shape on sectional plane extending parallel to the depth direction and there is a continuous transition from the channel wall to the boundary surface of the web-shaped element.

The film layers can be bonded to each other while also ensuring the drainage space producing the drainage effect by means of punctiform fixing areas preferably arranged in a grid pattern. Fixing can be performed by means of welding, gluing or other permanent types of bonding. However, this bonding should not impede the evacuation of exudates, but support it. At the same time, the application of a negative pressure to the wound space should also be promoted.

In this context, it has proven particularly beneficial if the individual fixing areas have an area of 5 mm² or less, in particular 3 mm², particularly preferably 2 mm² on a sectional plane extending perpendicular to the depth direction, wherein the spacing between the individual fixing areas or individual grid points of the fixing areas arranged in a grid pattern is 2 mm or more, in particular 3 mm or more, particularly preferably 5 mm or more. Regardless of whether fixing is performed with welding, gluing or other types of bonding, a material bridge connecting the internal boundary surfaces of the web-shaped elements to each other can be formed in the fixing areas.

To maintain the required capillary effect in the drainage space, the distance between the internal drainage areas of the film layers delimiting the drainage space is 5 mm or less, preferably 4 mm or less, particularly preferably 2 mm or less in the negative pressure treatment arrangements according to the invention. The capillary effect in the drainage space is thus ensured with the spacing between the internal faces of the web-shaped elements being 0.1mm or more, particularly preferably 0.3 mm or more, and optionally 0.04 mm or more.

In the case of negative pressure treatment arrangements according to the invention, at least one film layer comprises perforated plastic film, and in particular can generally be designed in the form of a perforated plastic film. To promote wound healing, it has proven particularly beneficial if at least one antibacterial or bacteriostatic material such as PHMB, silver, chlorhexidine or similar is provided which is preferably incorporated into at least one film layer and/or is provided in the form of a surface layer on at least one film layer. Furthermore, at least one film layer can include an agent counteracting the tendency of the drainage film to stick, such as a hydrophilic or hydrophobic fixture or a coating made from swellable materials.

As can be seen from the above explanation of negative pressure treatment arrangements according to the invention, the invention allows for the negative pressure treatment arrangement to have a device preferably incorporated into the drainage film, the fixing film and/or the absorbent body to produce a negative pressure in the area of the wound. This device could be a mechanical and/or electric pump that is connected to the wound space as applicable by means of a non-return valve in order to produce a negative pressure in the wound space once the negative pressure treatment arrangement is in place.

The invention also envisages the use of sensors to record the progress of treatment. To this end, the negative pressure treatment arrangement according to the invention can have at least one sensor preferably incorporated into the drainage film, the fixing film, the flange, the hose fitting, the drainage hose, the absorbent body and/or the device for producing a negative pressure to detect a temperature, a flow of fluid, a fluid viscosity or similar. In addition, sensors for recording the duration of treatment, the quantity of exudate evacuated, the pressure in the wound space or similar can also be provided.

In the framework of monitoring the therapy, it has proven particularly efficacious if the negative pressure treatment arrangement has associated therewith at least one transmission device to establish a wireless connection with an external device. In this way, data relevant to treatment can be transferred to a smartphone, a cloud or similar. Alarms, information about the charge level of a battery, where present, information about the fill level of an exudate receptacle, where present, and/or a suction body, information about the visual properties of the drainage film and/or fixing film or similar can thus be forwarded to an external device.

A negative pressure treatment arrangement according to the invention can be assembled as a finished assembly, taking account of the most common sizes and shapes. However, a negative pressure treatment arrangement according to the invention can also be provided as a modular system in which the individual components and layers are adapted to individual needs by the user. An individual drainage layer, cover layer, connection to a device producing a negative pressure (flange, drainage hose, etc.), antimicrobial substances or similar may be available in the modular system.

A negative pressure treatment kit according to the invention has a negative pressure treatment arrangement according to the invention and a device for producing a negative pressure in the drainage space and the wound space.

A negative pressure treatment arrangement according to the invention can be used to treat postoperative wound infections, in particular to treat contaminated laparotomy wounds.

## Claims

1. A negative pressure treatment arrangement comprising a multi-layered drainage film, wherein a drainage space is formed between two layers of the drainage film, a first film layer on the wound side delimiting the drainage space has a first opening arrangement facilitating the admission of fluids or gases to the drainage space and a second film layer facing away from the wound that also delimits the drainage space has a second opening arrangement designed to discharge fluids and/or gases from the drainage space, and comprising a fluid-tight fixing film that can be used to fix the drainage film to the skin surrounding a wound and to cover at least one area of the second opening arrangement, wherein the drainage film is made in some areas at least from a transparent material, whereby a vague profile of the covered wound at least is visible through the drainage film during negative pressure treatment, further comprising a connection arrangement for connecting a device for producing a negative pressure to the second opening arrangement comprising an open-cell sponge and/or a flange which can be placed on the second opening arrangement with a fitting for connecting a drainage hose, wherein the first opening arrangement has at least one channel starting from the first film layer, extending towards the opposite boundary surface of the other film layer and leading into the drainage space, the wall of which channel is integrally with the film layer, and is formed by perforating the film layer,
at least one opening arrangement comprises a plurality of openings preferably arranged in a grid pattern, wherein the spacing between adjacent openings is 15 mm or less, preferably 5 mm or less, in particular 3 mm or less, the spacing between the internal boundary surfaces of the film layers is 5 mm or less, in particular 4 mm or less, particularly preferably 2 mm or less and 0.1 mm or more, particularly preferably 0.3 mm or more and at least one film layer comprises a perforated plastic film, **characterised in that** the cross-sectional area of the channel is reduced on a plane extending perpendicular to the depth direction, starting from the first film layer and proceeding towards the opposite internal boundary surface.

2. The negative pressure treatment arrangement according to claim 1, **characterised in that** the connection arrangement can be fixed to the second film layer with the fixing film.

3. The negative pressure treatment arrangement according to one of the preceding claims, **characterised in that** the fixing film is made in some areas at least from a transparent material.

4. The negative pressure treatment arrangement according to one of the preceding claims, **characterised in that** the fixing film has a polyurethane film which is preferably coated with an adhesive such as a polyacrylate adhesive on one side at least.

5. The negative pressure treatment arrangement according to one of the preceding claims, **characterised by** a suction body for receiving and storing fluids from the wound.

6. The negative pressure treatment arrangement according to one of the preceding claims, **characterised in that** the depth of the drainage space in a depth direction extending perpendicular to the film layers ensures a capillary action on the bodily fluids received in the drainage area, such as exudates.

7. The negative pressure treatment arrangement according to claim any of the preceding claims, **characterised in that** the apertures of the openings located in one film layer are arranged in a projection along the depth direction between the apertures of the openings located in the other film layer.

8. The negative pressure treatment arrangement according to one of the preceding claims, **characterised in that** at least one channel extends over 50% or more of the entire depth of the drainage space in the depth direction.

9. The negative pressure treatment arrangement according to one of the preceding claims, **characterised in that** the aperture area of the individual openings facing the drainage space is 0.1 mm² or more, in particular 0.5 mm² or more, particularly preferably 1 mm² or more on a plane running perpendicular to the depth direction.

10. The negative pressure treatment arrangement according to one of the preceding claims, **characterised in that** the aperture area of the individual openings facing the drainage space is 15 mm² or less, preferably 5 mm² or less, in particular 4 mm² or less, particularly preferably 3 mm² or less.

11. The negative pressure treatment arrangement according to any of the preceding claims, **characterised in that** sections at least of the channel wall are designed in an arc shape on sectional plane extending parallel to the depth direction and there is a continuous transition to the boundary surface.

12. The negative pressure treatment arrangement according to one of the preceding claims, **characterised in that** at least one antimicrobial or bacteriostatic material such as PHMB, silver, chlorhexidine or similar is provided which is preferably incorporated into at least one film layer and/or is provided in the form of a surface layer on one film layer.

13. The negative pressure treatment arrangement according to one of the preceding claims, **characterised in that** at least one film layer includes an agent counteracting the tendency of the drainage film to stick, such as a hydrophilic or hydrophobic equipment or a coating made from swellable materials.

14. The negative pressure treatment arrangement according to one of the preceding claims, **characterised in that** an absorbent material such as foam, gauze or an open-cell material is coated between the film layers and/or a storage material such as a superabsorbent is provided between the film layers.

15. The negative pressure treatment arrangement according to one of the preceding claims, **characterised by** a device to produce a negative pressure in the area of the wound,preferably incorporated into the drainage film, the fixing film and/or the absorbent body.

16. The negative pressure treatment arrangement according to one of the preceding claims, **characterised by** at least one sensor preferably incorporated into the drainage film, the fixing film, the flange, the hose fitting, the drainage hose, the absorbent body and/or the device for producing a negative pressure, said sensor being operable to detect a temperature, a flow of fluid, a fluid viscosity or similar.

17. The negative pressure treatment arrangement according to one of the preceding claims, **characterised by** a transmission device for establishing a wireless connection with an external device.

18. A negative pressure treatment kit with a negative pressure treatment arrangement according to one of the preceding claims and a device for producing a negative pressure in the drainage space and the wound space.

## Patentansprüche

1. Unterdruckbehandlungsanordnung mit einer mehrlagigen Drainagefolie, wobei zwischen zwei Lagen der Drainagefolie ein Drainageraum gebildet ist, eine erste, den Drainageraum begrenzende wundseitige Folienlage eine erste den Eintritt von Flüssigkeiten oder Gasen in den Drainageraum ermöglichende Öffnungsanordnung aufweist und eine zweite, den Drainageraum ebenfalls begrenzende wundabgewandte Folienlage eine zweite zum Ableiten von Flüssigkeiten und/oder Gasen aus dem Drainageraum ausgelegte Öffnungsanordnung aufweist, und einer zum Fixieren der Drainagefolie an der eine Wunde umgebenden Haut und zum Abdecken zumindest eines Bereichs der zweiten Öffnungsanordnung einsetzbaren fluiddichten Fixierfolie, wobei die Drainagefolie zumindest bereichsweise aus einem transparenten Material besteht, wodurch während der Unterdruckbehandlung zumindest ein undeutliches Profil der abgedeckten Wunde durch die Drainagefolie hindurch sichtbar ist, ferner umfassend eine Anschlussanordnung zum Anschließen einer Einrichtung zum Erzeugen eines Unterdrucks an die zweite Öffnungsanordnung, welche Anschlussanordnung einen auf die zweite Öffnungsanordnung auflegbaren offenporigen Schwamm und/oder Flansch mit einem Anschlussstutzen zum Anschließen eines Drainageschlauchs aufweist, wobei die erste Öffnungsanordnung mindestens einen sich ausgehend von der ersten Folienlage in Richtung auf die gegenüberliegende Begrenzungsfläche der anderen Folienlage erstreckenden und in den Drainageraum mündenden Kanal aufweist, dessen Kanalwand vorzugsweise einstückig mit der Folienlage ausgeführt ist und durch Perforation der Folienlage gebildet ist,
wobei mindestens eine Öffnungsanordnung eine Vielzahl von vorzugsweise rasterförmig angeordneten Öffnungen aufweist, wobei der Abstand zwischen benachbarten Öffnungen 15 mm oder weniger, vorzugsweise 5 mm oder weniger, insbesondere 3 mm oder weniger beträgt, wobei der Abstand zwischen den inneren Begrenzungsflächen der Folienlagen 5 mm oder weniger, insbesondere 4 mm oder weniger, besonders bevorzugt 2 mm oder weniger und 0,1 mm oder mehr, besonders bevorzugt 0,3 mm oder mehr beträgt, und mindestens eine Folienlage eine perforierte Kunststofffolie aufweist, **dadurch gekennzeichnet, dass** die Querschnittsfläche des Kanals in einer sich senkrecht zur Tiefenrichtung erstreckenden Ebene ausgehend von der Folienlage in Richtung auf die gegenüberliegende innere Begrenzungsfläche verringert ist.

2. Unterdruckbehandlungsanordnung nach Anspruch 1, **dadurch gekennzeichnet, dass** die Anschlussanordnung mit der Fixierfolie an der zweiten Folienlage fixierbar ist.

3. Unterdruckbehandlungsanordnung nach einem der vorhergehenden Ansprüche, **dadurch gekennzeichnet, dass** die Fixierfolie zumindest bereichsweise aus einem transparenten Material ausgeführt ist.

4. Unterdruckbehandlungsanordnung nach einem der vorhergehenden Ansprüche, **dadurch gekennzeichnet, dass** die Fixierfolie eine Polyurethanfolie aufweist, die vorzugsweise zumindest einseitig mit einem Kleber wie Polyacrylatkleber beschichtet ist.

5. Unterdruckbehandlungsanordnung nach einem der vorhergehenden Ansprüche, **gekennzeichnet durch** einen Saugkörper zur Aufnahme und Speicherung von Flüssigkeiten aus der Wunde.

6. Unterdruckbehandlungsanordnung nach einem der vorhergehenden Ansprüche, **dadurch gekennzeichnet, dass** die Tiefe des Drainageraums in einer sich senkrecht zu den Folienlagen erstreckenden Tiefenrichtung eine Kapillarwirkung auf in dem Drainageraum aufgenommene Körperflüssigkeiten, wie etwa Exsudate, gewährleistet.

7. Unterdruckbehandlungsanordnung nach einem der vorhergehenden Ansprüche, **dadurch gekennzeichnet, dass** die Mündungen der in einer Folienlage angeordneten Öffnungen in einer Projektion längs der Tiefenrichtung zwischen den Mündungen der in der anderen Folienlage angeordneten Öffnungen angeordnet sind.

8. Unterdruckbehandlungsanordnung nach einem der vorhergehenden Ansprüche, **dadurch gekennzeichnet, dass** sich mindestens ein Kanal in Tiefenrichtung über 50 % oder mehr der gesamten Tiefe des Drainageraums erstreckt.

9. Unterdruckbehandlungsanordnung nach einem der vorhergehenden Ansprüche, **dadurch gekennzeichnet, dass** die dem Drainageraum zugewandte Mündungsfläche der einzelnen Öffnungen in einer senkrecht zur Tiefenrichtung verlaufenden Ebene 0,1 mm² oder mehr, insbesondere 0,5 mm² oder mehr, besonders bevorzugt 1 mm² beträgt.

10. Unterdruckbehandlungsanordnung nach einem der vorhergehenden Ansprüche, **dadurch gekennzeichnet, dass** die dem Drainageraum zugewandte Mündungsfläche der einzelnen Öffnungen 15 mm² oder weniger, vorzugsweise 5 mm² oder weniger, insbesondere 4 mm², besonders bevorzugt 3 mm² oder weniger beträgt.

11. Unterdruckbehandlungsanordnung nach einem der vorhergehenden Ansprüche, **dadurch gekennzeichnet, dass** die Kanalwand in einer sich parallel zur Tiefenrichtung erstreckenden Schnittebene zumindest abschnittweise bogenförmig ausgeführt ist und stetig in die Begrenzungsfläche übergeht.

12. Unterdruckbehandlungsanordnung nach einem der vorhergehenden Ansprüche, **dadurch gekennzeichnet, dass** mindestens ein antimikrobielles oder bakteriostatisches Material, wie etwa PHMB, Silber, Chlorhexidin oder dergleichen vorgesehen ist, das vorzugsweise in mindestens eine Folienlage eingearbeitet und/oder in Form einer Oberflächenschicht auf einer Folienlage vorgesehen ist.

13. Unterdruckbehandlungsanordnung nach einem der vorhergehenden Ansprüche, **dadurch gekennzeichnet, dass** mindestens eine Folienlage ein der Verklebungsneigung der Drainagefolie entgegenwirkendes Mittel, wie etwa eine hydrophile oder hydrophobe Ausstattung oder dem Auftrag von quellbaren Materialien, aufweist.

14. Unterdruckbehandlungsanordnung nach einem der vorhergehenden Ansprüche, **dadurch gekennzeichnet, dass** zwischen den Folienlagen ein Absorptionsmedium, wie etwa Schaum, Mull, oder ein offenporiges Material geschichtet ist und/oder ein Speichermaterial, wie etwa ein Superabsorber, zwischen den Folienlagen vorgesehen ist.

15. Unterdruckbehandlungsanordnung nach einem der vorhergehenden Ansprüche, **gekennzeichnet durch** eine Einrichtung zur Erzeugung eines Unterdrucks im Bereich der Wunde, vorzugsweise eingearbeitet in die Drainagefolie, die Fixierfolie und/oder den Saugkörper.

16. Unterdruckbehandlungsanordnung nach einem der vorhergehenden Ansprüche, **gekennzeichnet durch** mindestens einen vorzugsweise in die Drainagefolie, die Fixierfolie, den Flansch, den Schlauchstutzen, den Drainageschlauch, den Saugkörper und/oder die Einrichtung zur Erzeugung von Unterdruck eingearbeiteten Sensor, wobei der Sensor betreibbar ist, um eine Temperatur, eine Fluidströmung, eine Fluidviskosität oder ähnliches zu erfassen.

17. Unterdruckbehandlungsanordnung nach einem der vorhergehenden Ansprüche, **gekennzeichnet durch** eine Übertragungseinrichtung zum Herstellen einer drahtlosen Verbindung mit einem externen Gerät.

18. Unterdruckbehandlungskit mit einer Unterdruckbehandlungsanordnung nach einem der vorhergehenden Ansprüche und einer Einrichtung zum Erzeugen eines Unterdrucks in dem Drainageraum und dem Wundraum.

## Revendications

1. Ensemble de traitement par pression négative comprenant un film de drainage multicouche, dans lequel un espace de drainage est formé entre deux couches du film de drainage, une première couche du film située côté plaie et délimitant l'espace de drainage présente un premier ensemble d'ouvertures permettant l'entrée de fluides ou gaz dans l'espace de drainage et une deuxième couche du film détournée de la plaie et délimitant également l'espace de drainage présente un deuxième ensemble d'ouvertures conçu pour évacuer les fluides et/ou gaz de l'espace de drainage, et comprenant un film de fixation étanche aux fluides permettant de fixer le film de drainage à la peau entourant une plaie et de recouvrir au moins une zone du deuxième ensemble d'ouvertures, ledit film de drainage étant réalisé, au moins dans certaines zones, dans un matériau transparent, moyennant quoi la plaie recouverte est au moins vaguement visible à travers le film de drainage pendant le traitement par pression négative, comprenant en outre un ensemble de liaison qui est destiné à relier un dispositif de production de pression négative au deuxième ensemble d'ouvertures et qui comprend une éponge à pores ouverts susceptible d'être placée sur le deuxième ensemble d'ouvertures et/ou une bride susceptible d'être placée sur le deuxième ensemble d'ouvertures et dotée d'un raccord pour relier un tuyau de drainage, ledit premier ensemble d'ouvertures présentant au moins un canal partant de la première couche du film, s'étendant vers la surface limite opposée de l'autre couche du film et débouchant dans l'espace de drainage, la paroi dudit canal étant constituée d'un seul tenant avec la couche du film et étant formée par perforation de la couche du film,
au moins un ensemble d'ouvertures comprenant une pluralité d'ouvertures agencées de préférence en grille, l'écart entre des ouvertures adjacentes étant de 15 mm ou moins, de préférence de 5 mm ou moins, notamment de 3 mm ou moins, l'écart entre les surfaces limites internes des couches du film étant de 5 mm ou moins, notamment de 4 mm ou moins, tout particulièrement de 2 mm ou moins et de 0,1 mm ou plus, tout particulièrement de 0,3 mm ou plus et au moins une couche du film comprenant un film de plastique perforé, **caractérisé en ce que** la section transversale du canal est réduite sur un plan s'étendant perpendiculairement au sens de la profondeur, en partant de la première couche du film et en allant vers la surface limite interne opposée.

2. Ensemble de traitement par pression négative selon la revendication 1, **caractérisé en ce que** l'ensemble de liaison peut être fixé à la deuxième couche du film à l'aide du film de fixation.

3. Ensemble de traitement par pression négative selon l'une quelconque des revendications précédentes, **caractérisé en ce que** le film de fixation est réalisé, au moins dans certaines zones, dans un matériau transparent.

4. Ensemble de traitement par pression négative selon l'une quelconque des revendications précédentes, **caractérisé en ce que** le film de fixation présente un film de polyuréthane qui est de préférence enduit d'un adhésif tel qu'un adhésif polyacrylate sur au moins une face.

5. Ensemble de traitement par pression négative selon l'une quelconque des revendications précédentes, **caractérisé par** un corps aspirant destiné à recevoir et contenir des fluides issus de la plaie.

6. Ensemble de traitement par pression négative selon l'une quelconque des revendications précédentes, **caractérisé en ce que** la profondeur de l'espace de drainage, dans un sens de la profondeur perpendiculaire aux couches du film, assure une action capillaire sur les fluides corporels reçus dans la zone de trainage, tels que des exsudats.

7. Ensemble de traitement par pression négative selon l'une quelconque des revendications précédentes, **caractérisé en ce que** les embouchures des ouvertures situées dans une couche du film sont agencées en projection le long du sens de la profondeur entre les embouchures des ouvertures situées dans l'autre couche du film.

8. Ensemble de traitement par pression négative selon l'une quelconque des revendications précédentes, **caractérisé en ce qu'**au moins un canal s'étend sur 50 % ou plus de la profondeur totale de l'espace de drainage dans le sens de la profondeur.

9. Ensemble de traitement par pression négative selon l'une quelconque des revendications précédentes, **caractérisé en ce que** la surface d'embouchure des diverses ouvertures qui est tournée vers l'espace de drainage est de 0,1 mm² ou plus, notamment de 0,5 mm² ou plus, tout particulièrement de 1 mm² ou plus sur un plan perpendiculaire au sens de la profondeur.

10. Ensemble de traitement par pression négative selon l'une quelconque des revendications précédentes, **caractérisé en ce que** la surface d'embouchure des diverses ouvertures qui est tournée vers l'espace de drainage est de 15 mm² ou moins, de préférence de 5 mm² ou moins, notamment de 4 mm² ou moins, tout particulièrement de 3 mm² ou moins.

11. Ensemble de traitement par pression négative selon l'une quelconque des revendications précédentes, **caractérisé en ce que** des sections, au moins, de la paroi du canal sont conçues arquées sur un plan de coupe parallèle au sens de la profondeur et sont dans la continuité de la surface limite.

12. Ensemble de traitement par pression négative selon l'une quelconque des revendications précédentes, **caractérisé en ce qu'**au moins un matériau antimicrobien ou bactériostatique tel que du PHMB, de l'argent, de la chlorhexidine ou similaire est prévu en étant de préférence incorporé dans au moins une couche du film et/ou est prévu sous la forme d'une couche de surface présente sur une couche du film.

13. Ensemble de traitement par pression négative selon l'une quelconque des revendications précédentes, **caractérisé en ce qu'**au moins une couche du film comporte un agent inhibant la tendance du film de drainage à coller, tel qu'une garniture hydrophile ou hydrophobe ou un revêtement à base de matières dilatables.

14. Ensemble de traitement par pression négative selon l'une quelconque des revendications précédentes, **caractérisé en ce qu'**un matériau absorbant, tel que de la mousse, de la gaze ou une matière à pores ouverts, est revêtu entre les couches du film et/ou un matériau collecteur tel qu'un superabsorbant est prévu entre les couches du film.

15. Ensemble de traitement par pression négative selon l'une quelconque des revendications précédentes, **caractérisé par** un dispositif destiné à produire une pression négative à proximité de la plaie, de préférence incorporé dans le film de drainage, le film de fixation et/ou le corps absorbant.

16. Ensemble de traitement par pression négative selon l'une quelconque des revendications précédentes, **caractérisé par** au moins un capteur de préférence incorporé dans le film de drainage, le film de fixation, la bride, le raccord de tuyau, le tuyau de drainage, le corps absorbant et/ou le dispositif de production de pression négative, ledit capteur permettant de détecter la température, l'écoulement du fluide, la viscosité du fluide ou similaire.

17. Ensemble de traitement par pression négative selon l'une quelconque des revendications précédentes, **caractérisé par** un dispositif de transmission destiné à établir une connexion sans fil avec un appareil externe.

18. Trousse de traitement par pression négative comportant un ensemble de traitement par pression négative selon l'une quelconque des revendications précédentes et un dispositif de production de pression négative dans l'espace de drainage et l'espace de la plaie.
